# EUROPEAN PATENT SPECIFICATION

(11) **EP 1 237 600 B1**
(45) Date of publication and mention of the grant of the patent: **29.11.2006**
(21) Application number: 00988968.4
(22) Date of filing: 14.12.2000
(51) Int. Cl.: A61M 5/32

(54) **IMPROVEMENTS IN OR RELATING TO HYPODERMIC SYRINGES**
VERBESSERUNGEN BEZÜGLICH SUBKUTANINJEKTIONSSPRITZEN
AMELIORATIONS SE RAPPORTANT A DES SERINGUES HYPODERMIQUES

(30) Priority: 14.12.1999 GB 9929557
(43) Date of publication of application: 11.09.2002
(73) Proprietor: Parker, David William, Bury, Lancashire BL8 4QQ (GB); Burgess, Colin Hamilton, Ramsbottom, Lancashire BL0 9QR (GB)
(72) Inventor: Parker, David William, Bury, Lancashire BL8 4QQ (GB); Burgess, Colin Hamilton, Ramsbottom, Lancashire BL0 9QR (GB)
(74) Representative: Read, Matthew Charles
(86) International application number: PCT/GB2000/004815
(87) International publication number: WO 2001/043619

(56) References cited:
- EP-A- 0 505 330
- WO-A-00/18454
- US-A- 4 921 486
- US-A- 5 320 606
- US-A- 5 342 308
- US-A- 5 389 076
- US-A- 5 395 337
- US-A- 5 578 015

## Description

### Field of the invention

This invention relates to hypodermic syringes and particularly to a syringe in which the needle can be withdrawn.

### Background

Over recent years, the use of disposable syringes and needles has become increasingly dangerous. Although the risk of accidental scratch or puncture by a used needle, a so-called needle stick injury, has always existed, the increased risk of infection with, for example HIV or hepatitis has become a growing concern to all involved in the provision of health care.

It is estimated that in the USA, there are approximately 1,000,000 needle stick injuries annually which result in some 20,000 instances of infection with HIV or hepatitis. The consequent cost of these injuries is estimated at US$3 billion per annum. Hypodermic syringes with a retracting needle have been proposed, so that the needle is retracted after use to avoid needle stick injuries. For example US-A-5211628 discloses a hypodermic syringe with a housing, a plunger, a needle carrier with a needle mounted thereto, the needle carrier being mounted on the housing in a housing aperture, with the needle extending outwardly from the housing, and a stored energy configuration, the plunger and the stored energy configuration being arranged so that when the syringe is used and the plunger moves towards the needle carrier, it become attached thereto and the stored energy in the stored energy configuration is released to retract the needle carrier and the needle into the housing through the housing aperture.

In our WO 00/18454 there is described a disposable syringe in which the needle is fitted with a sheath that is attached to the needle carrier. The pre-sheathed needle is fitted into the housing aperture from within the housing thereby reducing the risk of needle injuries. A problem with this arrangement is that when the user wishes to remove the sheath, the needle carrier, which is circular in cross-section rotates within the housing aperture, making it difficult to remove the sheath by the usual twisting action.

A hypodermic syringe according to the preamble of claim 1 is known from document US-A-5 578 015.

Another problem arises if it is desired to make the needle detachable from the needle carrier. In a conventional syringe, the needle may be attached to the housing by a push fit. If the needle is push fitted to the movable needle carrier, to provide needle interchangeability, there is problem that the stored energy configuration, when withdrawing the needle carrier into the housing, may cause the needle to become detached from the needle carrier. It has been proposed in conventional syringes to securely attach a needle to the syringe using a so-called "Luer lock" which requires rotation of the needle axially relative to its mounting, so as to lock it in position. However, such a locking arrangement cannot be readily used with a retractable needle carrier because rotation of the needle will also produce rotation of the needle carrier relative to the housing making it difficult to secure engagement between them.

### Summary of the invention

The present invention provides a solution to this problem. In accordance with the present invention, the housing aperture and the needle carrier are configured so as not to rotate relative to one another about the longitudinal access of the needle.

Thus, a sheath may be mounted on the needle carrier surrounding the needle, so as to be removable from the carrier utilising an axial rotation of the sheath.

The needle may be interchangeably mounted and lockable onto the needle carrier by axial rotation of the needle relative to the carrier, for example utilising a Luer lock. Since the needle carrier is constrained from rotation relative to the housing, interchange of the needle on the needle carrier can be readily achieved according to the invention.

The housing aperture and the needle carrier may be configured so that the needle and sheath can be fitted within the housing.

The aperture and the co-acting portion of the needle carrier are non-circular and configured to permit withdrawal of the needle carrier into the housing on release of the stored energy configuration and to prevent relative axial rotation thereof. The co-acting portion of the needle carrier and the aperture may be rectangular in transverse cross-section.

A piston may be slidably mounted in the housing and releasably coupled to the plunger, the piston being operable when the plunger is moved towards the needle carrier to engage it such that the coupling between the piston and the plunger is released with the result that the stored energy configuration is released so as to retract the needle carried into the housing.

The stored energy configuration may comprise a vacuum which may be established between the piston and the plunger either internally during assembly of the syringe or by external means after or during assembly of the syringe. Alternative sources of stored energy may be used such as a spring.

Preferably, the housing aperture is sufficiently large to permit a bent needle to be withdrawn into the housing.

### Brief description of the drawings

In order that the invention may be more fully understood embodiments thereof will now be described by way of example with reference to the accompanying drawings in which:
Figure 1 is a schematic cross-section of a first embodiment of the invention;
Figure 2 is a schematic cross-section of a second embodiment of a syringe according to the invention;
Figure 3 is a schematic exploded illustration of the needle carrier and housing aperture in the syringes shown in Figures 1 and 2;
Figure 4A is an exploded view of a needle carrier and changeable needle and sheath arrangement according to a modification; and
Figures 4B and 4C are end views of the needle and needle carrier respectively.

### Detailed description

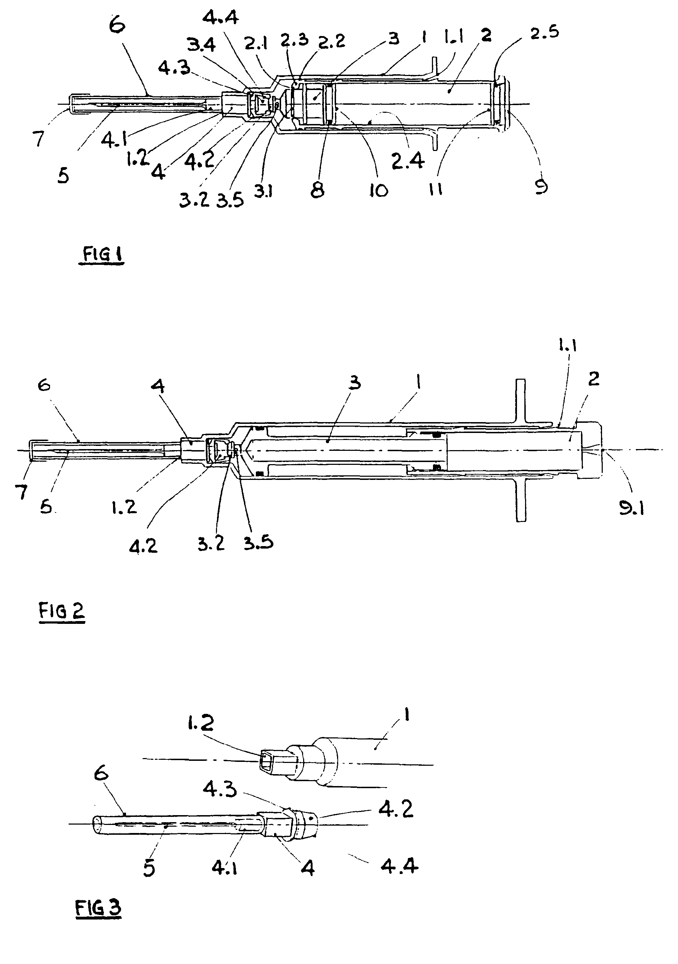

Referring to Figure 1, the syringe comprises a generally cylindrical housing 1 that has an open end 1.1 that receives a plunger 2 with an associated piston 3. The housing 1 contains a housing aperture 1.2 which receives the needle carrier 4 on which a needle 5 is mounted such that the longitudinal axis thereof is coaxial with the longitudinal axis of the housing 1 and slidable plunger 2. A sheath 6 with a removable end cap 7 is mounted on a coaxial flange 4.1 on the needle carrier 4.

In use, the plunger and piston arrangement is operated in a conventional manner to force an injectant through the needle 5 into a patient. Additionally, the syringe is provided with an automatic needle withdrawal mechanism as will now be explained.

The plunger 2 and piston 3 are assembled so as to create a vacuum in the interior space between them, which provides a stored energy configuration for withdrawing the needle, as will be explained later. To this end, the piston 3 is provided with a triangular sectioned ring 3.1 and the plunger is provided with a sealing surface 2.1 and a plunger head 2.2 which has a radially inwardly facing lip 2.3 that lodges against the triangular sectioned ring 3.1 to provide a releasable coupling. The piston 3 carries a seal 8 which slidably, sealingly engages the inner surface 2.4 of the plunger 2. A closure piece 9 on the plunger 2, is fixedly held in place by a wedge section ring 2.5 on the plunger.

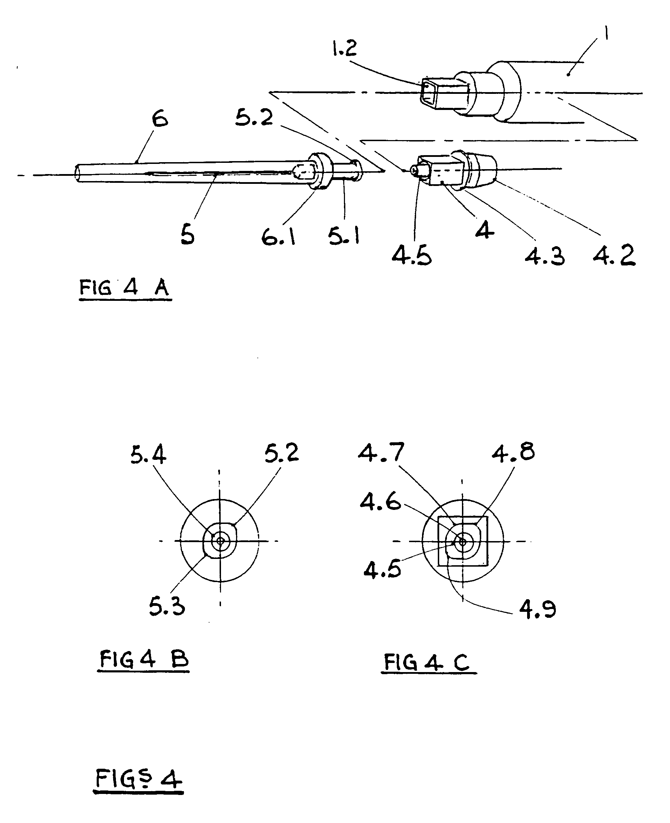

The needle carrier is formed with a cup 4.2, provided with an abutment lip 4.3, which is fixed onto the main body 4 of the needle carrier, as can be seen clearly in Figure 3. The cup 4.2 is provided with an annular lip 4.4 for receiving a head 3.2 on the piston. The piston head 3.2 is grooved to provide a by-pass 3.4 and includes a fluid passage 3.5.

In order to produce a vacuum between the plunger and the piston 3 during assembly, the piston 3 and the closure piece 9 are placed with their surfaces 10, 11 in contact with one another, so as to be held together using an appropriate lubricant. They are then inserted as one piece through the end of the plunger 2 and the closure piece 9 is inserted into groove 2.5. Thereafter, the piston 3 is slid axially along the plunger until the triangular section ring 3.1 snaps past the inward facing lip 2.3, thereby creating and maintaining a vacuum inside the plunger 2 and holding the piston 3 and plunger 2 together as once piece. The plunger closure 9 remains at the end of the plunger 2. The plunger and piston assembly is then ready for insertion into the housing 1.

However, before inserting the piston and plunger assembly, the assembly of the needle carrier 4, which includes the needle 5 together with sheath 6 and end cap 7, is inserted into the housing, so as to protrude through the housing aperture 1.2, with the abutment 4.3 engaging the interior of the aperture. The aperture 1.2 is sufficiently large to allow the passage of the needle 5, pre-shrouded by the protective sheath 6. The needle sheath and end cap 6, 7 thus affords protection to the needle 5 and also guidance when entering and locating the needle assembly in the housing apertures 1.2.

Operation of the syringe follows the established practice for disposable syringes.
On completion of an injection stroke, automatic needle retraction is triggered by continued pressure on the plunger 2 by the user. Referring to Figure 1, pressure on the plunger moves the plunger and the piston together as a single piece through the interior of the housing 1 towards the needle carrier 4, thereby expelling the injectant through the needle 5. When the piston 3 meets the end wall of the housing 1, the piston head 3.2 enters the cup 4.2 of the needle carrier 4 and is retained by the lip 4.4. The injectant fluid can then flow along the by-pass 3.4 and the fluid passage 3.5 thereby avoiding a fluid lock. Further inward movement of the piston 3 is now prohibited by the housing 1 and as a result, further inward pressure of the plunger 2 snaps the inward facing lip 2.3 past the triangular section ring 3.1, thereby releasing the piston 3 to slide relative to the plunger 2. As a result, the vacuum within the plunger 2 draws the piston 3 and the needle carrier 4 attached to it, into the plunger 2 thereby withdrawing the needle carrier 4 and the needle 5 automatically.

The pull exerted by the vacuum on the cup 4.2 during retraction elongates the cup 4.3 axially, thereby reducing its radial pressure against the interior wall of the housing 1 allowing free movement axially inwardly of the housing 1.

Referring now to Figure 3, it can be seen that the housing aperture 1.2 and the needle carrier 4 are square in cross-section such that when the needle carrier is received in the housing aperture 1.2 it is prevented from rotating about the axis of the needle 5. Thus, during assembly, when the needle carrier is inserted into the housing aperture 1.2 from within the housing 1, it is locked against rotation. In accordance with the invention, this has the advantage that when the user wishes to remove the sheath 6, this can be done by axially twisting the sheath so to remove it from the mounting region 4.1. Without this feature, the needle carrier would rotate, making it extremely difficult to remove the needle sheath without the risk of a needle stick injury.

Another embodiment of the invention is shown in Figure 2 wherein like parts are marked with the same reference numerals used in Figure 1. In this embodiment, the vacuum produced between the plunger 2 and piston 3 is created by using a vacuum pump or other source of vacuum that is coupled to orifice 9.1 so as to extract air from the interior space between the plunger 2 and piston 3. The orifice is thereafter sealed to maintain the vacuum. The plunger is operated as previously described with reference to Figure 1 so that when the piston head 3.2 engages the cup shaped member 4.2 on the needle carrier, further depression of the plunger breaks the releasable connection between the plunger 2 and piston 3, with the result that the vacuum withdraws the needle carrier 4 together with the needle 5 axially into the housing 1.

The structure of the housing aperture 1.2 and the needle carrier 4 is non-circular, as shown in Figure 3, so as to prevent axial rotation of the needle carrier relative to the housing 1.

It will be understood that many modifications and variations fall within the scope of the claimed invention. For example, instead of being rectangular, the needle carrier 4 and housing aperture 1.2 could be of other mutually co-operating non-circular shapes which prevent axial rotation of the needle carrier relative to the housing. It will be understood that the configuration eases the breaking of friction between the needle sheath 6 and the needle carrier 4 when the needle sheath is partially rotated, thus facilitating easier withdrawal of the needle sheath and consequently reducing the risk of damage and/or injury during this process.

Since the housing aperture 1.2 can be relatively large, the described examples of the invention have the capacity to retract a bent needle without it jamming against the side walls of the housing.

A modification to the syringe is shown in Figure 4, in which the needle is interchangeable. Like parts of those of Figures 1-3 are marked with the same reference numbers. Needle 5 is provided with a mounting nipple 5.1 with diametrically opposed end cams 5.2, 5.3. The nipple 5.1 which may be made of plastics material, is provided with an interior axial bore 5.4. The needle carrier 4 includes a central cylindrical spigot 4.5, which includes an axial bore 4.6 through which injectant is supplied to the needle 5. The spigot 4.5 is formed axially within a mounting recess 4.7 to receive the bore 5.4 of the nipple 5.1, the mounting recess including detents 4.8, 4.9 to receive the cams 5.2. 5.3. Thus, the needle can be mounted onto the needle carrier by aligning the cams 5.2, 5.3 with the corresponding detents 4.8, 4.9 and axially inserting the nipple 5.1 into the mounting recess 4.7. Then, by axially rotating the needle so as to axially rotate the cams 5.2, 5.3 and misalign them with the detents 4.8, 4.9, the needle 5 becomes locked into the needle carrier 4. The locking can be released by axially counter-rotating the needle 5 relative to the needle carrier 4 and subsequently withdrawing it in a reverse manner.

The needle sheath 6 is provided with a peripheral mounting ring 6.1 which may be frictionally engaged or otherwise releasably gripped onto the needle nipple 5.1. Thus, rotation of the needle 5 to lock it in place, can be achieved by gripping the needle sheath 6 and rotating the entire assembly. The needle can be removed in a reverse manner by gripping the sheath. Since the needle carrier 4 is rectangular in cross section and prevented from rotation by the corresponding shape of housing aperture 1.2, mounting and release of the needle from the needle carrier is readily facilitated in accordance with the invention. Furthermore, when the syringe is used, with the sheath removed, and the vacuum between plunger 2 and piston 3 is utilised to withdraw the needle carrier into the housing, the needle 5 is positively locked into the needle carrier 4, thereby minimising the risk of the needle becoming detached as a result of the withdrawal process. Thus, a so-called Lure lock can be provided between the needle and the needle carrier, such that the needle can be rotated to achieve the desired locking action, without corresponding rotation of the needle carrier 4.

Generally, it will be appreciated that the described embodiments of the invention exhibit the following advantageous features:
i) reliability and ease of use;
ii) automatic, complete and immediate retraction of the needle following injection;
iii) capacity to retract a bent needle;
iv) re-exposure of the needle is possible;
v) suitable for production in larger size with an off-set needle;
vi) supplied with the needle fitted and sheathed;
vii) suitable for supply pre-charged with an injectant;
viii) accidental needle retraction before injection is prevented
ix) low production costs;
x) firm and compact for safe disposal.

## Claims

1. A hypodermic syringe including a housing (1), a plunger (2), a needle carrier (4) with a needle (5) mounted thereto, the needle carrier (4) being mounted on the housing (1) in a housing aperture (1.2) with the needle (5) extending outwardly from the housing (1), and a stored energy configuration, the plunger (2) and the stored energy configuration being arranged so that when the syringe is used and the plunger (2) moves towards the needle carrier (4), it becomes attached thereto and the stored energy in the stored energy configuration is released to retract the needle carrier (4) and the needle (5) into the housing (1) through the housing aperture (1.2), **characterised in that** the housing aperture (1.2) and a co-acting portion of the needle carrier (4) have non-circular cross-sections which co-operate to prevent rotation relative to one another about the longitudinal axis of the needle (5).

2. A syringe according to claim 1 wherein the needle (5) is receivable onto the needle carrier (4) by axial rotation of the needle relative to the carrier.

3. A syringe according to claim 2 wherein a Luer lock is provided for locking the needle (5) onto the needle carrier (4).

4. A syringe according to any preceding claim including a sheath (6) mounted to the needle carrier (4) and surrounding the needle (5), separable from the carrier (4) by axial rotation of the sheath (6).

5. A syringe according to claim 4 wherein the housing aperture (1.2) and the needle carrier (4) are configured so that the needle (5) and sheath (6) can be fitted from within the housing (1).

6. A syringe according to any preceding claim wherein the aperture (1.2) and a co-acting portion of the needle carrier (4) are configured to permit withdrawal of the needle carrier (4) into the housing (1) on release of the stored energy configuration and to prevent relative axial rotation thereof.

7. A syringe according to claim 6 wherein the aperture (1.2) and a co-acting portion of the needle carrier (4) are rectangular in transverse cross section.

8. A syringe according to any preceding claim including a piston (3) slidably mounted in the housing (1) and releasably coupled to the plunger (2), the piston (3) being operable when the plunger (2) is moved towards the needle carrier (4) to engage it such that the coupling between the piston (3) and the plunger (2) is released and such that the stored energy configuration is released so as to retract the needle carrier (4) into the housing (1).

9. A syringe according to claim 8 wherein the stored energy configuration comprises a vacuum established between the piston (3) and the plunger (2).

10. A syringe according to claim 9 wherein the vacuum has been generated internally during assembly of the syringe.

11. A syringe according to claim 7 wherein the vacuum has been generated by external means during or after assembly of the syringe.

12. A syringe according to any preceding claim wherein the housing aperture (1.2) is sufficiently large to permit a bent needle to be withdrawn into the housing (1) by the release of the stored energy configuration.

## Patentansprüche

1. Injektionsspritze mit einem Gehäuse (1), einem Presskolben (2), einem Nadelträger (4) mit einer daran montierten Nadel (5), wobei der Nadelträger (4) in einer Gehäuseöffnung (1.2) am Gehäuse (1) montiert ist, wobei sich die Nadel (5) vom Gehäuse (1) nach außen erstreckt, und einer Energiespeichergestaltung, wobei der Presskolben (2) und die Energiespeichergestaltung derart eingerichtet sind, dass, wenn die Spritze benutzt wird und sich der Presskolben (2) in Richtung auf den Nadelträger (4) bewegt, er daran befestigt wird und die gespeicherte Energie in der Energiespeichergestaltung freigesetzt wird, um den Nadelträger (4) und die Nadel (5) durch die Gehäuseöffnung (1.2) in das Gehäuse (1) zurückzuziehen, **dadurch gekennzeichnet, dass** die Gehäuseöffnung (1.2) und ein zusammenwirkender Abschnitt des Nadelträgers (4) nicht-kreisförmige Querschnitte haben, welche zusammenwirken, um Drehung relativ zueinander um die Längsachse der Nadel (5) zu verhindern.

2. Spritze nach Anspruch 1, bei der die Nadel (5) durch axiale Drehung der Nadel relativ zum Träger auf dem Nadelträger (4) aufnehmbar ist.

3. Spritze nach Anspruch 2, bei der eine Luer-Verriegelung zum Verriegeln der Nadel (5) auf dem Nadelträger (4) vorgesehen ist.

4. Spritze nach irgendeinem vorhergehenden Anspruch, mit einer am Nadelträger (4) montierten und die Nadel (5) umgebenden Umhüllung (6), die durch axiale Drehung der Umhüllung (6) vom Träger (4) abtrennbar ist.

5. Spritze nach Anspruch 4, bei der die Gehäuseöffnung (1.2) und der Nadelträger (4) derart gestaltet sind, dass die Nadel (5) und Umhüllung (6) von innen am Gehäuse (1) angebracht werden können.

6. Spritze nach irgendeinem vorhergehenden Anspruch, bei der die Öffnung (1.2) und ein zusammenwirkender Abschnitt des Nadelträgers (4) derart gestaltet sind, dass Zurückziehen des Nadelträgers (4) in das Gehäuse (1) bei Auslösung der Energiespeichergestaltung erlaubt wird und relative axiale Drehung desselben verhindert wird.

7. Spritze nach Anspruch 6, bei der die Öffnung (1.2) und ein zusammenwirkender Abschnitt des Nadelträgers (4) rechtwinkligen Querschnitt haben.

8. Spritze nach irgendeinem vorhergehenden Anspruch, mit einem Kolben (3), der verschiebbar im Gehäuse (1) montiert ist und lösbar mit dem Presskolben (2) gekoppelt ist, wobei der Kolben (3) betätigbar ist, wenn der Presskolben (2) in Richtung auf den Nadelträger (4) bewegt wird, um derart daran anzugreifen, dass die Kopplung zwischen dem Kolben (3) und dem Presskolben (2) gelöst wird, und derart, dass die Energiespeichergestaltung ausgelöst wird, um den Nadelträger (4) in das Gehäuse (1) zurückzuziehen.

9. Spritze nach Anspruch 8, bei der die Energiespeichergestaltung ein zwischen dem Kolben (3) und dem Presskolben (2) hergestelltes Vakuum umfasst.

10. Spritze nach Anspruch 9, bei der das Vakuum während des Zusammenbaus der Spritze innen erzeugt worden ist.

11. Spritze nach Anspruch 7, bei der das Vakuum während oder nach dem Zusammenbau der Spritze durch äußere Mittel erzeugt worden ist.

12. Spritze nach irgendeinem vorhergehenden Anspruch, bei der die Gehäuseöffnung (1.2) so groß ist, dass eine verbogene Nadel durch die Auslösung der Energiespeichergestaltung in das Gehäuse (1) zurückgezogen werden kann.

## Revendications

1. Seringue hypodermique comprenant un corps (1), un plongeur (2), un support (4) d'aiguille sur lequel est montée une aiguille (5), le support (4) d'aiguille étant monté sur le corps (1) dans une ouverture (1.2) du corps de façon que l'aiguille (5) s'étende vers l'extérieur depuis le corps (1), et une configuration d'énergie emmagasinée, le plongeur (2) et la configuration d'énergie emmagasinée étant agencés de manière que, lorsque la seringue est utilisée et que le plongeur (2) se déplace vers le support (4) d'aiguille, il s'y attache et l'énergie emmagasinée dans la configuration d'énergie emmagasinée soit relâchée pour rétracter le support (4) d'aiguille et l'aiguille (5) à l'intérieur du corps (1) à travers l'ouverture (1.2) du corps, **caractérisée en ce que** l'ouverture (1.2) du corps et une partie coopérante du support (4) d'aiguille ont des sections transversales non circulaires qui coopèrent afin d'empêcher une rotation de l'une par rapport à l'autre autour de l'axe longitudinal de l'aiguille (5).

2. Seringue selon la revendication 1, dans laquelle l'aiguille (5) peut être reçue sur le support (4) d'aiguille par une rotation axiale de l'aiguille par rapport au support.

3. Seringue selon la revendication 2, dans laquelle un verrou Luer est prévu pour verrouiller l'aiguille (5) sur le support (4) d'aiguille.

4. Seringue selon l'une quelconque des revendications précédentes, comprenant un fourreau (6) monté sur le support (4) d'aiguille et entourant l'aiguille (5), pouvant être séparé du support (4) par une rotation axiale du fourreau (6).

5. Seringue selon la revendication 4, dans laquelle l'ouverture (1.2) du corps et le support (4) d'aiguille sont configurés de façon que l'aiguille (5) et le fourreau (6) puissent être ajustés depuis l'intérieur du corps (1).

6. Seringue selon l'une quelconque des revendications précédentes, dans laquelle l'ouverture (1.2) et une partie coopérante du support (4) d'aiguille sont configurées de façon à permettre un retrait du support (4) d'aiguille à l'intérieur du corps (1) lors d'une libération de la configuration d'énergie emmagasinée et à empêcher leur rotation axiale relative.

7. Seringue selon la revendication 6, dans laquelle l'ouverture (1.2) et une partie coopérante du support (4) d'aiguille ont une section transversale rectangulaire.

8. Seringue selon l'une quelconque des revendications précédentes, comprenant un piston (3) monté de façon coulissante dans le corps (1) et relié de façon amovible au plongeur (2), le piston (3) pouvant être manoeuvré lorsque le plongeur (2) est déplacé vers le support (4) d'aiguille afin de l'engager de façon que l'accouplement entre le piston (3) et le plongeur (2) soit libéré et la configuration d'énergie emmagasinée soit libérée pour rétracter le support (4) d'aiguille à l'intérieur du corps (1).

9. Seringue selon la revendication 8, dans laquelle la configuration d'énergie emmagasinée comprend un vide établi entre le piston (3) et le plongeur (2).

10. Seringue selon la revendication 9, dans laquelle le vide a été généré intérieurement pendant l'assemblage de la seringue.

11. Seringue selon la revendication 7, dans laquelle le vide a été généré par un moyen extérieur pendant ou après l'assemblage de la seringue.

12. Seringue selon l'une quelconque des revendications précédentes, dans laquelle l'ouverture (1.2) du corps est suffisamment grande pour permettre à une aiguille courbée d'être retirée à l'intérieur du corps (1) par la libération de la configuration d'énergie emmagasinée.
